# EUROPEAN PATENT APPLICATION

(11) **EP 3 936 600 A1**
(43) Date of publication of application: **12.01.2022**
(21) Application number: 20184321.6
(22) Date of filing: 06.07.2020
(51) Int. Cl.: C12M 1/00, C12M 1/26, C12M 1/36

(54) **AUTOMATED CENTRIFUGE SETUP OF A BIOPROCESSING INSTALLATION**

(71) Applicant: Sartorius Stedim Biotech GmbH, 37079 Göttingen (DE)
(72) Inventor: SABALLUS, Martin, 37075 Göttingen (DE); SCHOLZ, Jochen, 37077 Göttingen (DE)
(74) Representative: Gottschald Patentanwälte Partnerschaft mbB

(57) **Abstract**

The invention is directed to an automated centrifuge setup of a bioprocessing installation for the separation of a cell broth by centrifugation, wherein, for the centrifugation, the centrifuge setup comprises a centrifuge (1), wherein the centrifuge (1) comprises at least one centrifuge chamber (2.1) with a chamber inlet (3) and a chamber outlet (4), wherein the centrifuge setup comprises a liquid pumping arrangement (5) and a liquid network (6) with a number of liquid lines (7) communicating with the liquid pumping arrangement (5). It is proposed, that the centrifuge setup comprises a sensor arrangement (11) with at least one biomass sensor arrangement (12.1), which biomass sensor arrangement (12.1) is assigned to a liquid line (7) of the liquid network (6), that the process control (10) calculates an occurrence level, preferably a concentration, of biomass in the respective liquid line (7) based on the sensor signals of the biomass sensor arrangement (12.1) and that the process control (10) controls the valve arrangement (8) and/or the liquid pumping arrangement (5) during the loading cycle and/or the washing cycle and/or the discharging cycle based on the sensor signals of the sensor arrangement (11).

## Description

The present invention relates to an automated centrifuge setup of a bioprocessing installation according to the general portion of claims 1 and 14, a bioprocess installation with such a centrifuge setup according to claim 16 and a method for operating such a centrifuge setup according to claim 17.

The bioprocessing installation may be applied in various fields of bioprocessing technology. High efficiency in this field has been driven by the increasing demand for biopharmaceutical drugs. The efficiency in this sense is regarding not only the cost-effectiveness of the components to be used, but also the controllability of the processes connected thereto.

The known centrifuge setup (EP 2 310 486 B1), which is the starting point for the invention, comprises a number of centrifuge chambers, which are each assigned a chamber inlet and a chamber outlet. The centrifuge setup also comprises a liquid pumping arrangement assigned to the centrifuge and a liquid network for the transport of the liquid. Finally, the centrifuge setup comprises a process control for controlling at least the centrifuge, the liquid pumping arrangement and the valve arrangement.

The known centrifuge setup is designed as a fluidized bed centrifuge for performing a continuous centrifugation process. A loading cycle, during which cell broth is pumped into the chamber inlet, a washing cycle, during which a buffer is pumped into the chamber inlet, and a discharging cycle, during which a buffer is pumped to a chamber outlet are being performed based on a predetermined time pattern. This allows to run the process with a simple process control. However, there is room for improving the overall efficiency.

It is therefore the object of the invention, to improve the known centrifuge setup such that its operation efficiency and its process reliability is increased.

The above noted problem is solved by a centrifuge setup according to the general portion of claim 1 with the features of the characterizing portion of claim 1.

The general concept underlying the invention is the idea to use the sensor signals of a biomass sensor arrangement, which is assigned to a liquid line of the liquid network, for the control of the loading cycle and/or the washing cycle and/or the discharging cycle. With such a sensor based control of this part of the downstream process, the efficiency may be increased in view of various aspects. One aspect is the increase in efficiency regarding the extraction of the product, be it the supernatant or the cell harvest. Another aspect is the chance to run the process in an automated manner, involving less operator interventions leading to the aforementioned higher process reliability. Yet another aspect is the inline data processing of real time sensor signals, which generally is the basis for quick and oscillation free process control.

In detail it is proposed that the centrifuge setup comprises a sensor arrangement with at least one biomass sensor arrangement, which biomass sensor arrangement is assigned to a liquid line of the liquid network. The process control calculates an occurrence level, which may well be a concentration of biomass in the respective liquid line, based on the sensor signals of the biomass sensor arrangement.

The term "occurrence level" in this application generally is a variable, which represents the degree of occurrence of the respective entity, here and preferably of biomass, in the respective liquid line. This variable may represent a continuous range between "occurrence" and "no occurrence". This is for example the concentration of the respective entity within the liquid in the liquid line. The occurrence level may also represent a binary information being either "occurrence" or "no occurrence" of the entity in the liquid line. As will be shown later, the term "occurrence level" may well be applied to the supernatant within the respective liquid line as well.

It is essential now that the process control controls the valve arrangement and/or the pumping arrangement during the loading cycle and/or the washing cycle and/or the discharging cycle based on the sensor signals of the sensor arrangement.

The proposed inline measurement of biomass makes it possible to control the valve arrangement and/or the pumping arrangement based on real time information which increases the preciseness of the control and with it the overall efficiency of the process.

Besides the at least one biomass sensor arrangement, according to claim 2, the sensor arrangement may comprise at least one supernatant sensor arrangement, which allows to determine an occurrence level of supernatant in the respective liquid line. This gives a chance to react on the change in liquid in the respective liquid line not only in view of the existence of biomass, but also on the existence of supernatant.

Claim 3 further defines the loading cycle, the washing cycle and the discharging cycle, assigning those cycles respective fluid lines.

Claims 4 and 5 are directed to using the sensor signals of the biomass sensor arrangement during the loading cycle by comparing a biomass filling level, that has been calculated based on the sensor signals of the biomass sensor arrangement, with a maximum biomass filling level. With this it is possible to extend the loading cycle as long as possible without the risk of overloading the centrifuge chamber.

Claims 6 and 7 are directed to using the sensor signals of the supernatant sensor arrangement also during the loading cycle. Here the goal is to find the optimal switching point for switching between the outlet supernatant line and, for example, the outlet waste line in order to waste as little supernatant as possible.

Claims 8 and 9 are directed to using the sensor signals of the biomass sensor arrangement for the control of the discharging cycle. Here, the goal is to find the optimal switching point for switching between the inlet harvest line and, for example, the inlet waste line, in order to waste as little cell harvest as possible.

The preferred embodiments according to claims 10 to 13 are directed to advantageous basic designs of the biomass sensor arrangement on the one hand and the supernatant sensor arrangement on the other hand. According to claim 11, the biomass sensor arrangement comprises a biomass sensor, which is realized as a capacitance biomass sensor. This may easily be realized with high sensor dynamics and low costs. The same is to be said for the preferred realization of the supernatant sensor according to claim 11, according to which the supernatant sensor is realized as a conductivity sensor.

A particularly compact and at the same time simple arrangement is subject of claim 13, according to which two sensor electrodes do not only provide a capacitance biomass sensor, but as well a conductivity sensor for the detection of supernatant.

A second independent teaching according to claim 14 is directed to an automated centrifuge setup as such, for which the existence of a biomass sensor arrangement is preferred, but not crucial. According to this teaching, it is most important that the centrifuge setup comprises a sensor arrangement with at least one supernatant sensor arrangement, which is assigned to a liquid line of the liquid network, and that the supernatant sensor arrangement comprises a supernatant sensor, which is realized as a conductivity sensor. Also with such a sensor based control of the respective part of the downstream process, the efficiency may be increased in view of various aspects as noted above.

A particularly preferred embodiment is the combination of a biomass sensor and a supernatant sensor in one single sensor according to a variant of claim 15. As noted above, this embodiment goes along with a compact and at the same time simple arrangement

A third independent teaching according to claim 16 is directed to a bioprocess installation with a proposed centrifugation setup and with a cell broth source in the form of a production vessel, in particular a bioreactor, or a storage vessel. All explanations given with regard to the first and second teaching are fully applicable to this third teaching.

A fourth independent teaching according to claim 17 is directed to a method for operating a centrifuge setup, wherein the centrifuge setup comprises a sensor arrangement with at least one biomass sensor arrangement, which biomass sensor arrangement is assigned to a liquid line of the liquid network. The proposed method represents the operation of the centrifuge setup according to the first teaching, such that, here as well, all explanations are given with regard to the first teaching, are fully applicable to this third teaching.

Claims 18 to 20 are each directed to preferred embodiments regarding the operation during the loading cycle and the discharging cycle. Again, all explanations given with regard to the first teaching are fully applicable.

In the following, a preferred embodiment of the invention is being described with regard to the drawings. In the drawings show
- Fig. 1: a schematic view on a proposed centrifuge setup during the loading cycle,
- Fig. 2: a schematic view on the centrifuge setup according to Fig. 1 during the washing cycle,
- Fig. 3: a schematic view on the centrifuge setup according to Fig. 1 during the discharging cycle,
- Fig. 4: a flowchart representing the steps of the loading cycle,
- Fig. 5: a flowchart representing the steps of the washing cycle, and
- Fig. 6: a flowchart representing the steps of the discharging cycle.

The proposed centrifuge setup, here and preferably, is assigned to the downstream process of a bioreactor or the like, processing a liquid in the form of a cell broth. Accordingly, the wording "liquid" is to be understood in a broad sense. It includes not only liquids as such, but also solutions and suspensions with particles like cells, cell debris, etc.

This centrifuge setup may be operated in different operating modes. A first operating mode, which is the focus of the present invention, is the clarification of the cell broth from any solid particles by centrifugation and subsequent filtration. The goal here is to separate the cell broth from solid particles such as cells, cell debris, etc., which solid particles are considered as biomass in the following. The product to be obtained in this first operating mode is the supernatant of the cell broth containing a product of interest such as an antibody or another pharmaceutical compound.

A second operating mode, which may generally be realized by the present invention as well, is the separation of cells in the cell broth from supernatant. The product to be obtained in this second operating mode are the cells in the cell broth.

For the centrifugation, the centrifuge set up comprises a centrifuge 1, wherein the centrifuge 1 comprises at least one centrifuge chamber 2.1, 2.2, 2.3, 2.4, each with a chamber inlet 3 and a chamber outlet 4. In the drawings, the altogether four centrifuge chambers 2.1, 2.2, 2.3, 2.4 are indicated. In this particular case, the chamber inlets 3 of the centrifuge chambers 2.1, 2.2, 2.3, 2.4 are connected with each other and the chamber outlets 4 of the centrifuge chambers 2.1, 2.2, 2.3, 2.4 are connected with each other to act as a single chamber inlet respective a single chamber outlet from the outside.

Here and preferably, each chamber 2.1, 2.2, 2.3, 2.4 is located offset a centrifuge rotor axis, wherein, further preferably, the respective chamber inlet 3 is located further away from the centrifuge rotor axis than the respective chamber outlet 4.

The expression "chamber inlet" means that the liquid to be centrifuged enters the respective chamber 2.1, 2.2, 2.3, 2.4 via the respective chamber inlet 3. The expression "chamber outlet" means that the centrifuged liquid exits the respective chamber 2.1, 2.2, 2.3, 2.4 via the respective chamber outlet 4. This is only to be understood as a definition of the fluid interface of the respective chamber 2.1, 2.2, 2.3, 2.4. As will be explained later, in certain situations, the chamber inlets 3 may be used as outlets and the chamber outlets 4 may be used as inlets respectively.

In order to facilitate the explanation of the invention, in the following, the specification states only one centrifuge chamber 2.1 with the chamber inlet 3 and the chamber outlet 4. All explanations regarding this centrifuge chamber 2.1 are fully applicable to any other centrifuge chamber, which may be provided.

The centrifuge setup comprises a liquid pumping arrangement 5 assigned to the centrifuge 1 and a liquid network 6 with a number of liquid lines 7 communicating with the fluid pumping arrangement 5, wherein the centrifuge setup comprises a valve arrangement 8 that allows to deactivate and activate at least one of the liquid lines 7, preferably by closing and opening the respective valve 9 of the valve arrangement 8. Accordingly, the valves 9 of the valve arrangement 8 are located within or at least at one end of the respective liquid lines 7 to be activated and deactivated.

The centrifuge setup is further designed to execute a loading cycle, during which cell broth is pumped into the chamber inlet 3, a washing cycle, during which a buffer is pumped into the chamber inlet 3, and a discharging cycle, during which a buffer is pumped into the chamber outlet 4.

In addition the centrifuge setup comprises a process control 10 for controlling at least the centrifuge 1, the liquid pumping arrangement 5 and the valve arrangement 8. Here it is to be pointed out that at least part of the valves 9 of the valve arrangement 8 are controllable by the process control 10.

It is now essential for the invention that the centrifuge setup comprises a sensor arrangement 11 with at least one biomass sensor arrangement 12.1, which biomass sensor arrangement 12.1 is assigned to a liquid line 7 of the liquid network 6, wherein the process control 10 calculates an occurrence level, here and preferably a concentration, of biomass in the respective liquid line 7 based on the sensor signals of the biomass sensor arrangement 12.1. It is finally essential for the invention that the process control 10 controls the valve arrangement 8 and/or the liquid pumping arrangement 5 during the loading cycle and/or the washing and/or the discharging cycle based on the sensor signals of the sensor arrangement 11.

Here and preferably, the centrifuge 1 is designed as a fluidized bed centrifuge for performing a continuous centrifugation process. The preferred setup of the centrifuge 1 is described in European Patent EP 2 485 846 B1.

The centrifuge 1 comprises a rotor, which may be rotated around the centrifuge rotor axis by a preferably electric motor, which is controlled by the process control 10 for the realization of a centrifuge revolution speed. For centrifugation, the liquid pumping arrangement 5 pumps cell broth through the centrifuge chamber 2.1.
The centrifuge revolution speed as well as the pumping rate are adjusted, preferably by the process control 8, with the aim to establish a fluidized bed of particles such as cells or cell debris in the centrifuge chamber 2.1. A fluidized bed is achieved when the centrifugal force on a particle is equal to the opposing fluid flow force so that a zero net force is exerted on the particle.

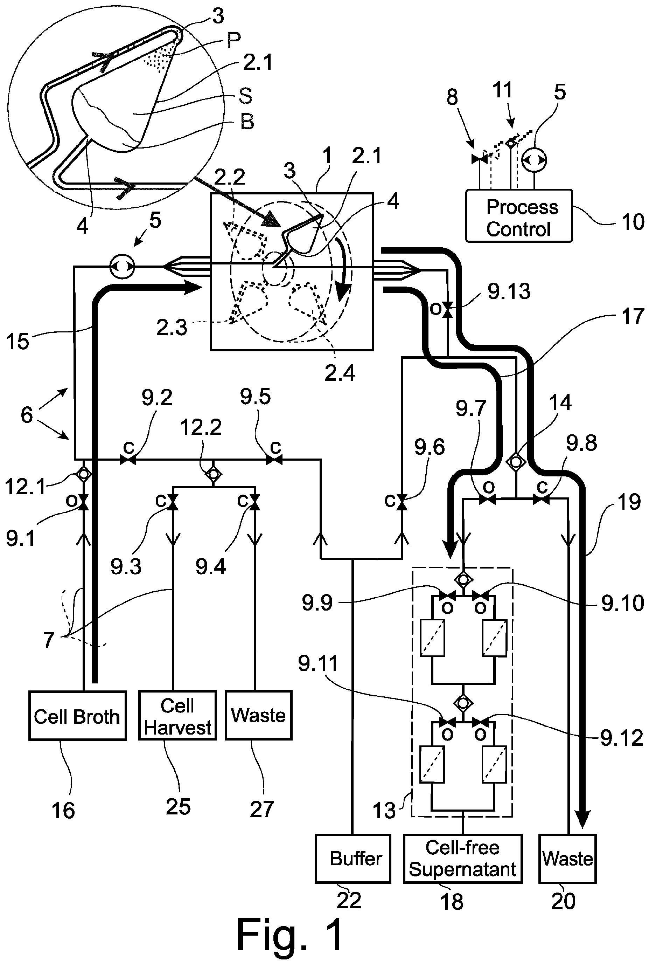

In case the product to be obtained is a particle free supernatant, the centrifuged cell broth is pumped through a filter arrangement 13 of the centrifuge setup as shown in Fig. 1. Here and preferably, the valve arrangement 8 comprises at least one valve 9, here and preferably valves 9.1 - 9.13, controlled by the process control 10, that allows to deactivate and activate at least one of the liquid lines 7, preferably by closing and opening the respective valve 9. In the drawings, the valves 9.1 - 9.13 are assigned the status "c" for closed and "o" for opened.

For better understanding, in the detailed view of the centrifuge chamber 2.1 in Fig. 1, the buffer is indicated with reference "B", the supernatant is indicated with reference "S" and the particles are indicated with reference "P".

According to the proposed solution, it is intended that the valve arrangement 8 is controllable by the process control 10 as noted above, such that activation and deactivation of the respective liquid lines 7 may be easily automated. The term "activate" and "deactivate" with regard to the liquid lines 7 generally means that liquid flow through the respective liquid line 7 is enabled or disabled, in particular blocked.

The above noted process control 10 may be realized as a central unit controlling all or at least most of the components of the centrifuge setup. The process control 10 may also be realized in a decentralized structure, comprising a number of decentralized units. In any case, the process control 10 comprises at least one microprocessor, on which a software may be run.

The sensor arrangement 11 preferably comprises at least one supernatant sensor arrangement 14, which is assigned to a liquid line 7 of the liquid network 6, wherein the process control 10 calculates an occurrence level, preferably a concentration, of supernatant in the respective liquid line 7 based on the sensor signals of the supernatant sensor arrangement 14.

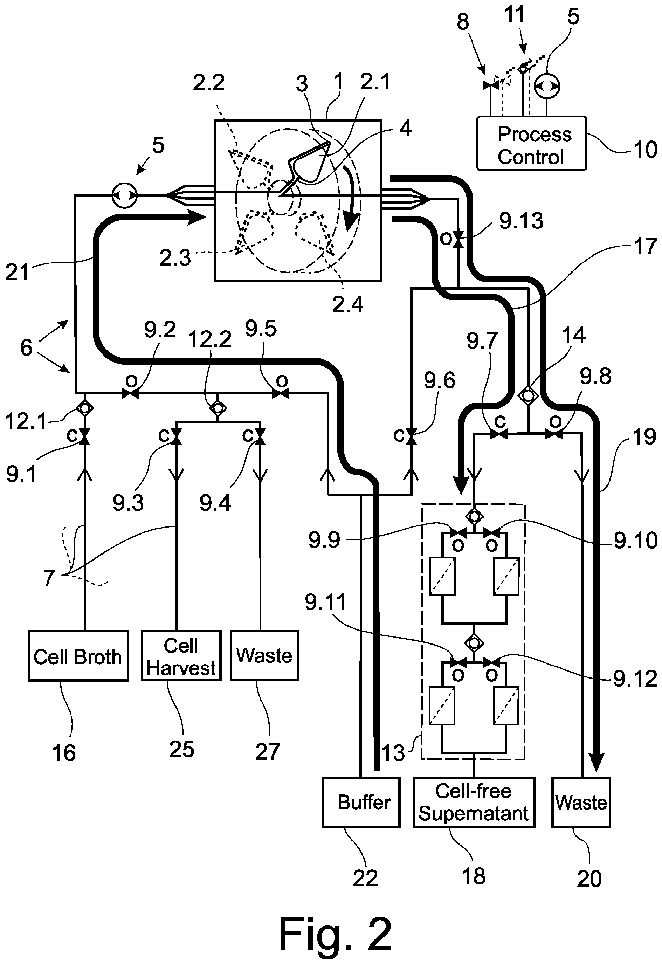

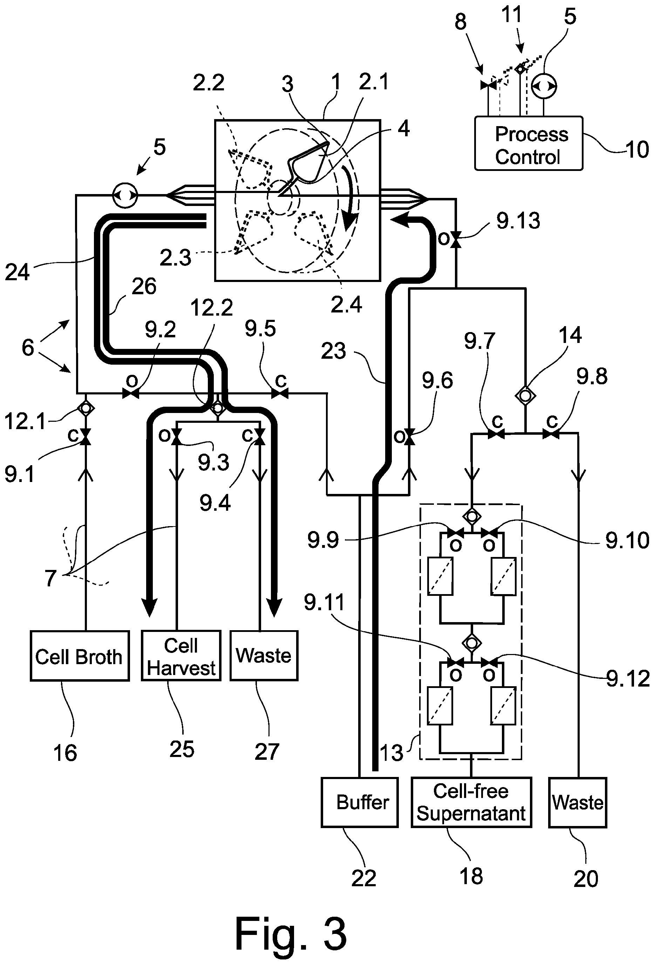

As noted above, the centrifuge setup may be operated in a loading cycle, which is indicated in Fig. 1, in a washing cycle, which is indicated in Fig. 2 and in a discharging cycle, which is indicated in Fig. 3. Accordingly, the liquid network 6 comprises an inlet feed line 15 between the chamber inlet 3 and a cell broth source 16, preferably a production or a storage vessel, and an outlet supernatant line 17 between the chamber outlet 4 and a supernatant reception 18, preferably a supernatant vessel. As an alternative or in addition, there may be an outlet waste line 19 between the chamber outlet and a waste reception 20.

Here it is to be understood, that for example the outlet supernatant line 17 and the outlet waste line 19 overlap each other along a certain liquid line section. Accordingly, the outlet supernatant line 17 on the one hand and the outlet waste line 19 on the other hand do not have to be separate from each other along their complete extent. This is true for all other definitions of liquid lines presented here, which are being provided by part of the liquid network 6.

During a loading cycle, the cell broth may be pumped by the liquid pumping arrangement 5 from the cell broth source 16 to the chamber inlet 3 via the inlet feed line 15, while the supernatant is flowing from the chamber outlet 4 to the supernatant reception 18 via the outlet supernatant line 17 or from the chamber outlet 4 to a waste reception 20 via the outlet waste line 19. This is indicated in Fig. 1.

Fig. 2 indicates, that the liquid network 6 comprises an inlet buffer line 21 between the chamber inlet 3 and a buffer source 22 and an outlet waste line 19 between the chamber outlet 4 and a waste reception 20, preferably a waste vessel, and that during a washing cycle the buffer may be pumped by the liquid pumping arrangement 5 from the buffer source 22 to the chamber inlet 3 via the inlet buffer line 21 and from the chamber outlet 4 to the waste reception 20 via the outlet waste line 19 or from the chamber outlet 4 to the supernatant reception 18 via the outlet supernatant line 17.

Fig. 3 indicates, that the liquid network 6 comprises an outlet buffer line 23 between the chamber outlet 4 and a buffer source 22 and an inlet cell harvest line 24 between the chamber inlet 3 and a cell harvest reception 25, preferably a cell harvest vessel, or an inlet waste line 26 between the chamber inlet 3 and a waste reception 27.

During a discharging cycle the buffer may be pumped from the buffer source 22 to the chamber outlet 4 via the outlet buffer line 23, while the buffer including solid particles, preferably cell harvest, is flowing from the chamber inlet 3 to the cell harvest reception 25 via the inlet cell harvest line 24 or from the chamber inlet 3 to a waste reception 27 via the inlet waste line 26.

In a preferred embodiment, the sensor signals of the biomass sensor arrangement 12.1 are used to determine a biomass filling level of the centrifuge chamber 2.1. The term "biomass filling level" means a value, that represents the amount of biomass in the form of solid particles having been filled into the centrifuge chamber 2.1. In case, the biomass sensor arrangement 12.1, which is located upstream the centrifuge chamber 2.1 during loading, not only comprises a biomass sensor for determining the biomass concentration, but also a liquid flow sensor, the resulting biomass volume in the centrifuge chamber 2.1 may easily be calculated. Alternatively, the biomass filling level of a centrifuge chamber 2.1 can easily be determined by use of a pre-calibrated pump 5 located upstream of the centrifuge chamber 2.1 which has a known pump loading flow rate.

In a further preferred embodiment, a maximum biomass filling level is defined in the process control 10, which shall be undercut by the calculated biomass filling level to prevent overloading. In an easy to realize and preferred variant, the maximum biomass filling level is determined based on an estimation or an empirical evaluation, which maximum amount of biomass may be fitted into the inside volume of the centrifuge chamber 2.1. In control view, an easy to realize variant is the normalization of the calculated biomass filling level relative to the maximum biomass filling level.

In detail, a biomass sensor arrangement 12.1 of the sensor arrangement 11 is located in the inlet feed line 15, wherein, during the loading cycle, the process control 10 calculates a biomass filling level of the centrifuge chamber 2.1 based on the sensor signals of the biomass sensor arrangement 12.1. As noted above, preferably, a maximum biomass filling level is defined in the process control 10 wherein, during the loading cycle, the process control 10 terminates the loading cycle, when the maximum biomass filling level is reached by the calculated biomass filling level.

In addition, preferably, the above noted supernatant sensor arrangement 14 is located in the outlet supernatant line 17 or the outlet waste line 19 wherein, during the loading cycle, the process control 10 calculates an occurrence level of supernatant in the outlet supernatant line 17 or the outlet waste line 19 based on the sensor signals of the supernatant sensor arrangement 14.

The sensor signals of the supernatant sensor arrangement 14 is useful in particular during the beginning of the loading cycle, which goes along with buffer in the centrifuge chamber 2.1, and during the washing cycle, which goes along with supernatant in the centrifuge chamber 2.1. In both cases, here and preferably, the supernatant is to be considered the product of interest. In the first case, a switch from the outlet waste line 19 to the outlet supernatant line 17 is necessary, when the remaining buffer has been pumped out of the centrifuge chamber 2.1, in order to prevent dilution of the supernatant within the supernatant reception 18. In the second case, a switch from the outlet supernatant line 17 to the outlet waste line 19 is necessary, when the remaining supernatant has been pumped out of the centrifuge chamber 2.1, again in order to prevent dilution of the supernatant within the supernatant reception 18.

The above noted, two cases may be detected based on the sensor signals of the supernatant sensor arrangement 14. For this, at least one switching level is to be defined in the process control 10. In the first above noted case, this supernatant switching level indicates, when the supernatant reaches the chamber outlet 4, which goes along with the buffer being pumped out of the centrifuge chamber 2.1. In the second above noted case, the supernatant switching level indicates, when the supernatant is pumped out of the centrifuge chamber 2.1.

In detail, an above noted supernatant switching level is defined in the process control 10 wherein, during the loading cycle and/or the washing cycle, the process control 10 switches between the outlet supernatant line 17 and the outlet waste line 19, when the supernatant switching level is exceeded or undercut by the occurrence level of supernatant calculated based on the sensor signals of the supernatant sensor arrangement 14. It may also be advantageous, that, during the loading cycle and/or the washing cycle, the process control 10 switches between the outlet supernatant line 17 and another liquid line not displayed, or terminates the loading cycle respective the washing cycle, when the supernatant switching level is exceeded or undercut by the occurrence level of supernatant calculated based on the sensor signals of the supernatant sensor arrangement 14.

In case, the cell harvest is to be considered the product of interest, again the sensor signals of a biomass sensor arrangement 12.2 of the sensor arrangement 11 are particularly helpful. This is in order to ensure, that during the discharging cycle, after the cells have been pumped out of the centrifuge chamber 2.1, no buffer is unduly pumped into the cell harvest reception 25.

Accordingly, the centrifuge setup preferably comprises a, here and preferably additional, biomass sensor arrangement 12.2 of the sensor arrangement 11 in the inlet cell harvest line 24 or the inlet waste line 26. During the discharging cycle, the process control 10 calculates an occurrence level of biomass in the inlet cell harvest line 24 or the inlet waste line 26 based on the sensor signals of this additional biomass sensor arrangement 12.2. For this, at least one biomass switching level is defined.

The additional biomass sensor arrangement 12.2 may well be provided by the biomass sensor arrangement 12.1 noted above, if the biomass sensor arrangement 12.1 would, for example, be arranged next to the liquid pumping arrangement 5 in Fig. 1.

In detail, preferably, an above noted biomass switching level is defined in the process control 10, wherein, during the discharging cycle, the process control 10 switches between the inlet cell harvest line 24 and the inlet waste line 26, when the biomass switching level is exceeded or undercut by the occurrence level of biomass calculated based on the sensor signals of the biomass sensor arrangement 12.2. It may also be advantageous, that, during the discharging cycle, the process control 10 switches between the inlet cell harvest line 24 and another liquid line, or terminates the discharging cycle, when the biomass switching level is exceeded or undercut by the occurrence level of biomass calculated based on the sensor signals of the biomass sensor arrangement 12.2.

In a compact arrangement, the biomass sensor arrangement 12.1, 12.2 comprises a biomass sensor and a flow sensor in combination.

The biomass sensor arrangement 12.1, 12.2 comprises a biomass sensor, which is preferably realized as a capacitance biomass sensor. The capacitance biomass sensor derives an occurrence level of biomass based on the dielectric properties, in particular permittivity, of the liquid in the respective liquid line 7.

The supernatant sensor arrangement 14 on the other hand, preferably comprises a supernatant sensor, which is realized as a conductivity sensor. It is further preferred, that the conductivity sensor derives an occurrence level of supernatant based on the conductivity of the liquid in the respective liquid line 7.

A particularly integrated and thereby compact design may be achieved, if the biomass sensor and the supernatant sensor are provided by a combined sensor with at least two sensor electrodes, wherein the combined sensor derives an occurrence level of biomass based on a capacitance measurement using the electrodes and that the combined sensor derives an occurrence level of supernatant based on a conductivity measurement using the electrodes.

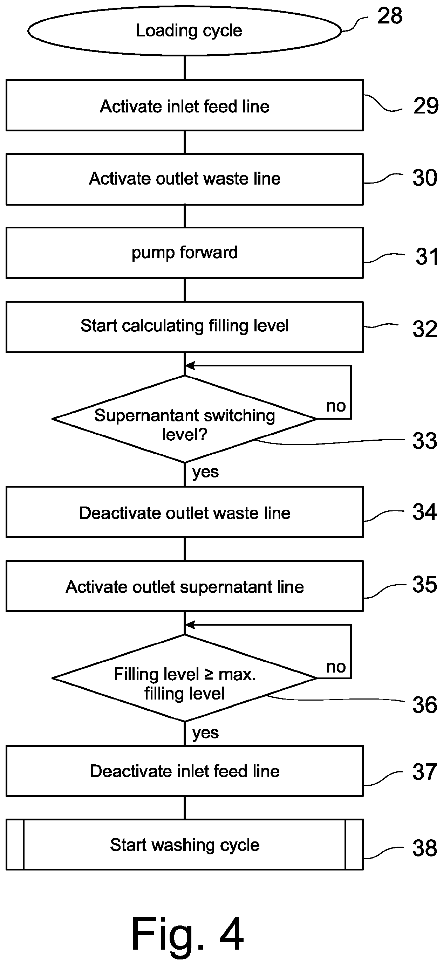

In view of the additional teachings regarding a bioprocess installation and a method for operating a proposed centrifuge setup, reference is made to the explanations given above. However, the method will be explained in even more detail with regard to fig. 4 to 6.

The flowchart shown in Fig. 4 is representing the loading cycle, which start is indicated with reference number 28. After activating the inlet feed line 15 by opening valve 9.1 in step 29 and the outlet waste line 19 by opening valves 9.8, and 9.13 in step 30, the liquid pumping arrangement 5 is operated in the forward direction in step 31. In step 32, the biomass filling level in the centrifuge chamber 2.1 is calculated based on the sensor signals of the biomass sensor arrangement 12.1, which is assigned to the inlet feed line 15. By this pumping action, the buffer, which is remaining in the centrifuge chamber 2.1 due to the previous discharging cycle or the initial buffer priming of the chamber, is being pumped out of the centrifuge chamber 2.1 to the waste reception 20 via the outlet waste line 19. This is done, until in step 33, it has been detected by the supernatant sensor arrangement 14, that the supernatant switching level has been reached. If so, the outlet waste line 34 is deactivated by closing valves 9.8 and 9.13 in step 34 and the outlet supernatant line 17 is activated by opening valves 9.7, 9.9 and/or 9.10, 9.11 and/or 9.12, and 9.13 in step 35. This results in the supernatant being pumped to the supernatant reception 18 via the outlet supernatant line 17 and the filter arrangement 13. If in step 36 it has been detected, that the calculated biomass filling level exceeded the maximum biomass filling level or a pre-defined biomass filling level defined in the process control 10, the inlet feed line 15 is deactivated by closing the valve 9.1 in step 37, and the washing cycle is executed in step 38.

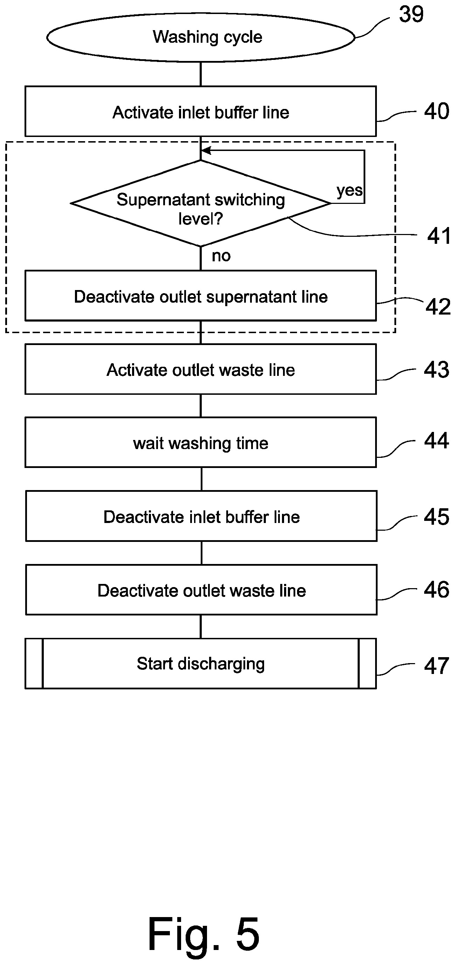

The flowchart in Fig. 5 shows the washing cycle, starting with step 39. First of all, the inlet buffer line 21 is activated by opening valves 9.2 and 9.5 in step 40 such that the buffer is pumped still in the forward direction to the supernatant reception 18 via the outlet supernatant line 17 and the filter arrangement 13. This is done until, in step 41, it has been detected based on the sensor signals of the supernatant sensor arrangement 14, that the supernatant switching level has been reached, in which case the outlet supernatant line 17 is deactivated by closing valves 9.7, 9.9 and/or 9.10, 9.11 and/or 9.12, respectively, in step 42 and activating outlet waste line 19 by opening valves 9.8 in step 43, such that the buffer is pumped to the waste reception 20. After having waited a determined washing time in step 44, the inlet buffer line 21 is deactivated by closing the valves 9.2, 9.5 in step 45 and the outlet waste line 19 is deactivated by closing valves 9.8, 9.13 in step 46. Here and preferred, the washing time is determined by the supernatant switching level. After that, the discharging cycle is being executed in step 47.

It may be noted, that the steps 41 and 42 may be omitted, if the supernatant is not the product of interest, such that the supernatant, which is remaining in the centrifuge chamber 2.1 from the previous loading cycle, does not have to be saved. This is indicated by the dotted box in Fig. 5.

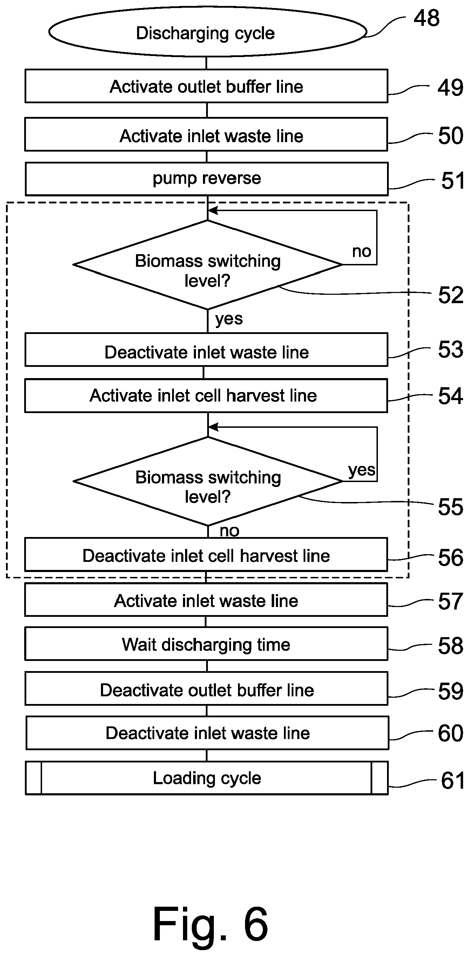

The flowchart in Fig. 6 shows the discharging cycle, starting with step 48. After activating the outlet buffer line 23 by opening valves 9.6, 9.13 in step 49 and activating the inlet waste line 26 by opening valves 9.2, 9.4 in step 50, the liquid pumping arrangement 5 is being reversed in step 51, such that the buffer remaining in the centrifuge chamber 2.1 from the previous washing cycle is pumped to the waste reception 27, until it has been detected in step 52 based on the biomass sensor arrangement 12.2, that the biomass switching level has been reached. Then, the inlet waste line 26 is deactivated by closing valve 9.4 in step 53 and the inlet cell harvest line 24 is activated by opening valve 9.3 in step 54. If the biomass switching level is undercut again in step 55, the inlet cell harvest line 24 is deactivated by closing valve 9.3 in step 56 and the inlet waste line 26 is activated by opening valve 9.4 in step 57. After having waited a predetermined discharging time in step 58, the outlet buffer line 23 is deactivated by closing valves 9.6, 9.13 in step 59 and the inlet waste line 26 is deactivated by closing valves 9.2, 9.4 in step 60.

Subsequently, the loading cycle is executed again in step 61, such that the periodic overall process continues.

It may be pointed out, that the steps 52 to 56 may be omitted, if the cell harvest is not the product of interest. This is indicated by the box in dotted lines in Fig. 6

Finally, it may be summed up, that with the proposed solution, the overall efficiency of the operation of the centrifuge setup may be increased, in particular by increasing the precision for switching the valves 9 of the valve arrangement based on the sensor signals of the sensor arrangement 11. This is realized by the process control 10 being in control wise connection with at least part of the valves 9 of the valve arrangement 8.

## Claims

1. Automated centrifuge setup of a bioprocessing installation for the separation of a cell broth by centrifugation, wherein, for the centrifugation, the centrifuge setup comprises a centrifuge (1), wherein the centrifuge (1) comprises at least one centrifuge chamber (2.1) with a chamber inlet (3) and a chamber outlet (4), wherein the centrifuge setup comprises a liquid pumping arrangement (5) and a liquid network (6) with a number of liquid lines (7) communicating with the liquid pumping arrangement (5),
wherein the centrifuge setup comprises a valve arrangement (8), that allows to activate and deactivate at least one of the liquid lines (7), preferably by closing and opening the respective valve (9) of the valve arrangement (8),
wherein the centrifuge setup is designed to execute a loading cycle, during which cell broth is pumped to the chamber inlet (3), preferably a washing cycle, during which a buffer is pumped to the chamber inlet (3), and a discharging cycle, during which a buffer is pumped to the chamber outlet (4) and
wherein the centrifuge setup comprises a process control (10) for controlling at least the centrifuge (1), the liquid pumping arrangement (5) and the valve arrangement (8),
**characterized in**
**that** the centrifuge setup comprises a sensor arrangement (11) with at least one biomass sensor arrangement (12.1), which biomass sensor arrangement (12.1) is assigned to a liquid line (7) of the liquid network (6), that the process control (10) calculates an occurrence level, preferably a concentration, of biomass in the respective liquid line (7) based on the sensor signals of the biomass sensor arrangement (12.1) and that the process control (10) controls the valve arrangement (8) and/or the liquid pumping arrangement (5) during the loading cycle and/or the preferred washing cycle and/or the discharging cycle based on the sensor signals of the sensor arrangement (11).

2. Centrifuge setup according to claim 1, **characterized in that** the sensor arrangement (11) comprises at least one supernatant sensor arrangement (14), which is assigned to a liquid line (7) of the liquid network (6), and that the process control (10) calculates an occurrence level, preferably a concentration of supernatant in the respective liquid line (7) based on the sensor signals of the supernatant sensor arrangement (14).

3. Centrifuge setup according to claim 1 or 2, **characterized in that** the liquid network (6) comprises an inlet feed line (15) between the chamber inlet (3) and a cell broth source (16), preferably a production vessel, in particular a bioreactor or storage vessel, and an outlet supernatant line (17) between the chamber outlet (4) and a supernatant reception (18), preferably a supernatant vessel, and/or an outlet waste line (19) between the chamber outlet (4) and a waste reception (20) and that during a loading cycle the cell broth may be pumped by the liquid pumping arrangement (5) from the cell broth source (16) to the chamber inlet (3) via the inlet feed line (15), while the supernatant is flowing from the chamber outlet (4) to the supernatant reception (18) via the outlet supernatant line (17) or from the chamber outlet (4) to a waste reception (20) via the outlet waste line (19), and/or, that the liquid network (6) comprises an inlet buffer line (11) between the chamber inlet (3) and a buffer source (22) and an outlet waste line (19) between the chamber outlet (4) and a waste reception (20), preferably a waste vessel, and that during a preferred washing cycle the buffer may be pumped by the liquid pumping arrangement (5) from the buffer source (22) to the chamber inlet (3) via the inlet buffer line (21) and from the chamber outlet (4) to the waste reception (20) via the outlet waste line (19) or from the chamber outlet (4) to the supernatant reception (18) via the outlet supernatant line (17), and/or, that the liquid network (6) comprises an outlet buffer line (23) between the chamber outlet (4) and a buffer source (22) and an inlet cell harvest line (24) between the chamber inlet (3) and a cell harvest reception (25), preferably a cell harvest vessel, or an inlet waste line (26) between the chamber inlet (3) and a waste reception (27), and that during a discharging cycle the buffer may be pumped from the buffer source (22) to the chamber outlet (4) via the outlet buffer line (23), while the buffer including solid particles, preferably cell harvest, is flowing from the chamber inlet (3) to the cell harvest reception (25) via the inlet cell harvest line (24) or from the chamber inlet (3) to a waste reception (27) via the inlet waste line (26).

4. Centrifuge setup according to any one of the preceding claims, **characterized in that** a biomass sensor arrangement (12.1) is located in the inlet feed line (15) and that, during the loading cycle, the process control (10) calculates a biomass filling level of the centrifuge chamber (2.1) based on the sensor signals of the biomass sensor arrangement (12.1).

5. Centrifuge setup according to claim 4, **characterized in that** a maximum biomass filling level is defined in the process control (10) and that, during the loading cycle, the process control (10) terminates the loading cycle, when the maximum biomass filling level is reached by the calculated biomass filling level.

6. Centrifuge setup according to any one of the preceding claims, **characterized in that** the supernatant sensor arrangement (14) is located in the outlet supernatant line (17) or the outlet waste line (19) and that, during the loading cycle, the process control (10) calculates an occurrence level of supernatant in the outlet supernatant line (17) or the outlet waste line (19) based on the sensor signals of the supernatant sensor arrangement (14).

7. Centrifuge setup according to claim 6, **characterized in that** a supernatant switching level is defined in the process control (10) and that, during the loading cycle and/or the washing cycle, the process control (10) switches between the outlet supernatant line (17) and the outlet waste line (19) or another liquid line, or terminates the loading cycle respective the washing cycle, when the supernatant switching level is exceeded or undercut by the occurrence level of supernatant calculated based on the sensor signals of the supernatant sensor arrangement (14).

8. Centrifuge setup according to any one of the preceding claims, **characterized in that** the centrifuge setup comprises a biomass sensor arrangement (12.2) of the sensor arrangement (11) in the inlet cell harvest line (24) or the inlet waste line (26) and that, during the discharging cycle, the process control (10) calculates an occurrence level of biomass in the inlet cell harvest line (24) or the inlet waste line (26) based on the sensor signals of the biomass sensor arrangement (12.2).

9. Centrifuge setup according to any one of the preceding claims, **characterized in that** a biomass switching level is defined in the process control (10) and that, during the discharging cycle, the process control (10) switches between the inlet cell harvest line (24) and the inlet waste line (26) or another liquid line, or terminates the discharging cycle, when the biomass switching level is exceeded or undercut by the occurrence level of biomass calculated based on the sensor signals of the biomass sensor arrangement (12.2).

10. Centrifuge setup according to any one of the preceding claims, **characterized in that** the biomass sensor arrangement (11) comprises a biomass sensor and a flow sensor in combination.

11. Centrifuge setup according to any one of the preceding claims, **characterized in that** the biomass sensor arrangement (12.1) comprises a biomass sensor, which is realized as a capacitance biomass sensor, preferably, that the capacitance biomass sensor derives an occurrence level of biomass based on the dielectric properties, in particular the permittivity, of the liquid in the respective liquid line (7).

12. Centrifuge setup according to any one of the preceding claims, **characterized in that** the supernatant sensor arrangement (14) comprises a supernatant sensor, which is realized as a conductivity sensor, preferably, that the conductivity sensor derives an occurrence level of supernatant based on the conductivity of the liquid in the respective liquid line (7).

13. Centrifuge setup according to any one of the preceding claims, **characterized in that** the biomass sensor and the supernatant sensor are provided by a combined sensor with at least two sensor electrodes, and that the combined sensor derives an occurrence level of biomass based on a capacitance measurement using the electrodes and that the combined sensor derives an occurrence level of supernatant based on a conductivity measurement using the electrodes.

14. Automated centrifuge setup of a bioprocessing installation for the separation of a cell broth by centrifugation, wherein, for the centrifugation, the centrifuge setup comprises a centrifuge (1), wherein the centrifuge (1) comprises at least one centrifuge chamber (2.1) with a chamber inlet (3) and a chamber outlet (4), wherein the centrifuge setup comprises a liquid pumping arrangement (5) and a liquid network (6) with a number of liquid lines (7) communicating with the liquid pumping arrangement (5),
wherein the centrifuge setup comprises a valve arrangement (8), that allows to activate and deactivate at least one of the liquid lines (7), preferably by closing and opening the respective valve (9) of the valve arrangement (8),
wherein the centrifuge setup is designed to execute a loading cycle, during which cell broth is pumped to the chamber inlet (3), preferably a washing cycle, during which a buffer is pumped to the chamber inlet (3), and a discharging cycle, during which a buffer is pumped to the chamber outlet (4) and
wherein the centrifuge setup comprises a process control (10) for controlling at least the centrifuge (1), the liquid pumping arrangement (5) and the valve arrangement (8),
**characterized in**
**that** the centrifuge setup comprises a sensor arrangement (11) with at least one supernatant sensor arrangement (14), which is assigned to a liquid line (7) of the liquid network (6), that the supernatant sensor arrangement (14) comprises a supernatant sensor, which is realized as a conductivity sensor, and that the process control (10) controls the valve arrangement (8) and/or the liquid pumping arrangement (5) during the loading cycle and/or the preferred washing cycle and/or the discharging cycle based on the sensor signals of the sensor arrangement (11).

15. Centrifuge setup according to claim 14, **characterized in that** the sensor arrangement (11) comprises at least one biomass sensor arrangement (12.1), which biomass sensor arrangement (12.1) is assigned to a liquid line (7) of the liquid network (6), preferably, that the biomass sensor arrangement (12.1) comprises a biomass sensor, which is realized as a capacitance biomass sensor, further preferably, that the biomass sensor and the supernatant sensor are provided by a combined sensor with at least two sensor electrodes, and that the combined sensor derives an occurrence level of biomass based on a capacitance measurement using the electrodes and that the combined sensor derives an occurrence level of supernatant based on a conductivity measurement using the electrodes.

16. Bioprocess installation with a centrifuge setup according to any one of the preceding claims and with a cell broth source (16) in the form of a production vessel, in particular a bioreactor, or a storage vessel.

17. Method for operating a centrifuge setup according to any one of the claims 1 to 15 or a bioprocess installation according to claim 16, wherein the centrifuge setup comprises a sensor arrangement (11) with at least one biomass sensor arrangement (12.1), which biomass sensor arrangement (12.1) is assigned to a liquid line (7) of the liquid network (6), that the process control (10) calculates an occurrence level, preferably a concentration, of biomass in the respective liquid line based on the sensor signals of the biomass sensor arrangement (12.1) and that the valve arrangement (8) and/or the liquid pumping arrangement (5) is/are being controlled by the process control (10) during the loading cycle and/or the washing cycle and/or the discharging cycle based on the sensor signals of the sensor arrangement (11).

18. Method according to claim 17, **characterized in that** a maximum biomass filling level is defined in the process control (10) and that, during the loading cycle, the loading cycle is being terminated by the process control (10), when the maximum biomass filling level is reaches by the calculated biomass filling level.

19. Method according to claim 17 or 18, **characterized in that** a supernatant switching level is defined in the process control (10) and that, during the loading cycle and/or the washing cycle, the process control (10) switches between the outlet supernatant line (17) and the outlet waste line (19), when the supernatant switching level is exceeded or undercut, and/or,
that a supernatant switching level is defined in the process control (10) and that, during the washing cycle, the process control (10) terminates the washing cycle and initiates the discharging cycle, when the supernatant switching level is exceeded or undercut.

20. Method according to any one of the claims 17 to 19, **characterized in that** a biomass switching level is defined in the process control (10) and that, during the discharging cycle, the process control (10) switches between the inlet harvest line (24) and the inlet waste line (26), when the biomass switching level is exceeded or undercut.
